# EUROPEAN PATENT SPECIFICATION

(11) **EP 1 835 881 B1**
(45) Date of publication and mention of the grant of the patent: **04.04.2012**
(21) Application number: 05767954.0
(22) Date of filing: 26.07.2005
(51) Int. Cl.: A61K 6/10, A61C 9/00

(54) **WAXES DIRECTLY APPLICABLE TO THE ORAL CAVITY DURING THE PREPARATION PHASE FOR CROWNS OR FIXED DENTAL BRIDGES**
DIREKT IN DE MUNDHÖHLE APPLIZIERBARE WACHSE ZUM EINSATZ WÄHREND DER HERSTELLUNGSPHASE FÜR KRONEN ODER FIXIERTE ZAHNBRÜCKEN
CIRES DIRECTEMENT APPLICABLES DANS LA CAVITE BUCCALE DURANT LA PHASE DE PREPARARTION DE COURONNES ET BRIDGES DENTAIRES FIXES

(30) Priority: 19.11.2004 IT MI20042229
(43) Date of publication of application: 26.09.2007
(73) Proprietor: Tralee Trading e Servicos LDA, 9000 Funchal-Madeira (PT)
(72) Inventor: LANCELLOTTI, Paolo, I-20034 Giussano (IT); SORMANI, Margherita, I-20034 Giussano (IT); MARZORATI, Alberto, I-20100 Milano (IT)
(74) Representative: Gervasi, Gemma
(86) International application number: PCT/IB2005/002174
(87) International publication number: WO 2006/054132

(56) References cited:
- DATABASE WPI Section Ch, Week 198204 Derwent Publications Ltd., London, GB; Class A96, AN 1982-07269E XP002367714 & SU 820 816 B (KHARK MED PLASTIC STOMAT) 25 April 1981 (1981-04-25)
- GAN H W: "Alternative simplifying method for construction of dental prosthesis directly from bite" J JPN PROSTHODONT SOC (NIPPON HOTETSU SHIKA GAKKAI ZASSHI), vol. 33, no. 3, 1989, pages 724-727, XP008060069
- DATABASE WPI Section PQ, Week 200143 Derwent Publications Ltd., London, GB; Class P32, AN 2001-401364 XP002367715 & JP 2001 112790 A (NISSAN SYSTEM KAIHATSU KK) 24 April 2001 (2001-04-24)
- ITO MICHIO; KUROIWA AKIHIRO; NAGASAWA SAKAE; YOSHIDA TAKAMITSU; YAGASAKI HIROSHI; OSHIDA YOSHIKI: "Effect of wax melting range and investment liquid concentration on the accuracy of a three-quarter crown casting" THE JOURNAL OF PROSTHETIC DENTISTRY, vol. 87, no. 1, January 2002 (2002-01), pages 57-61, XP002367710 US
- DIWAN ET AL: "Pattern waxes and inaccuracies in fixed and removable partial denture castings" JOURNAL OF PROSTHETIC DENTISTRY, XX, XX, vol. 77, no. 5, May 1997 (1997-05), pages 553-555, XP005143285 ISSN: 0022-3913

## Description

### Field of invention

This invention regards waxes that can be directly applied to the oral cavity during the preparation phase for fixed and/or combined crowns or dental bridges.

### Prior art

Fixed prosthesis work is carried out on stumped teeth (reduced in size) or on implanted posts (endosseus implants).

The professional makes two impressions.
- the first involves the dental arch on which the fixed prosthesis work will be carried out (in materials such as polyvinyl siloxanes or polyethers)
- the second involves the antagonist dental arch (in materials such as alginate) The two impressions that arrive at the dental technician's laboratory are filled with plaster and then mounted on an articulator.

Wax is used to model the missing teeth and the teeth on the stumps, thus creating the definitive shape of the article, and then a silicone template is made which will used to model the metal framework which supports the aesthetic material.

The silicone template is used to obtain the exact shape of the tooth, since the metal framework must be shaped so that a layer of aesthetic material can be applied to it which is as evenly thick as possible all over the surface.

The thickness of the aesthetic material will determine the appearance and the colour of the tooth.

Several problems may arise during this phase, as there are many parameters to take into consideration, such as for example the proportions of the occlusion of the arches, the correct sizes and the shapes of the teeth.

In the prior art, in order to check these parameters, resin is applied to the metal framework using the mask produced previously; the model of the tooth produced in this way is then tried out on the patient in the so-called "metal test", in other words a test to see how precise the metal framework is. The weak point of this procedure lies in the fact that most of the work has already been carried out and, if the metal test shows that the tooth being tested does not fulfil all the necessary requirements, all the work has to be started again.

In SU820816 is disclosed a composition for taking a cast for a one-piece dental crown, said composition having a needle penetration of 6.5-7 mm is suitable for obtaining high quality impression.

Gan H.W. in J. Jpn. Prosthodont. Soc. 1989, 33, 724-727 discloses a method for the construction of a dental prosthesis simplifying the occlusal adjustment procedure by taking the impression of the bite pattern directly from the bite. Inlay wax and paraffin wax were used to take functional bite pattern.

JP2001/112760 discloses pigmented dental wax materials (one white and the other black) which have moderate plasticity (with easy work with a spatula, a knife, etc., heat solubility, and the shape maintenance nature at the time of cooling) and have improved reflection/absorption efficiency when irradiated by a laser beam. The therein disclosed waxes are used out of the patient's mouth on a gypsum fibrosum model: the white wax is used for corking the air-bubbles eventually present in the gypsum model or to repair a broken model, the black wax is used to mark the lower margin of the anchor tooth in order to be unequivocally read by the laser bean. The two waxes are therefore useful for their reliable reflecting/absorbing properties when irradiated by a laser beam when designing a dental crown by CAD/CAM system.

Therefore, it was felt that there was the need to devise a system that would make it possible to avoid the aforementioned drawbacks, which would also conveniently reduce the time need for workmanship and as a result, the associated costs.

### Summary of the invention

The Applicant has unexpectedly found that it is possible to avoid the aforementioned drawbacks by using, during the preparation phase of fixed and/or combined crowns or dental bridges on both natural stumps and implanted posts, waxes that can be applied to the patient's mouth and then be functionalized.

These waxes have the following characteristics:
□ solidification point between 60 and 90°C, measured according to standard ASTM D938,
□ needle penetration measured according to standard ASTM D1321 between 8 and 20 dmm (tenths of mm),
□ kinematic viscosity measured at 99°C according to standard ASTM D445 between 6 and 20 mm²/s.

These waxes can be used specifically for preparing models of teeth for tests in the patient's mouth, without using the bridge or the tooth in metal with resin supports. Therefore, a further objective of this invention is a model for a test in the mouth, using the aforementioned waxes.

### Description of the figures

Figures 1-4 show phase (a) in the preparation of crowns and dental bridges with the traditional technique, using waxes according to this invention.
Figures 5 and 6 show phase (d) in the preparation of crowns and dental bridges with the traditional technique, using waxes according to this invention.
Figures 7 and 8 show phase (e1) in the preparation of crowns and dental bridges with the traditional technique.
Figures 9 and 10 show phase (e2) in the preparation of crowns and dental bridges with the traditional technique, using waxes according to this invention.
Figures 11-16 show phase (e3) in the preparation of crowns and dental bridges with the traditional technique, using waxes according to this invention.
Figures 17-21 show phase (a') in the preparation of crowns and dental bridges according to the PRESS OVER technique, using waxes according to this invention.

### Detailed description of the invention

In this description :
□ polymerization means hardening following the reticulation of the resin,
□ shelled teeth are defined as the teeth that will be subject to prosthesis,
□ functionalization of the waxes means the testing of the masticatory, aesthetic and phonetic functions carried out directly on the teeth modelled in wax, before starting work on the definitive models.

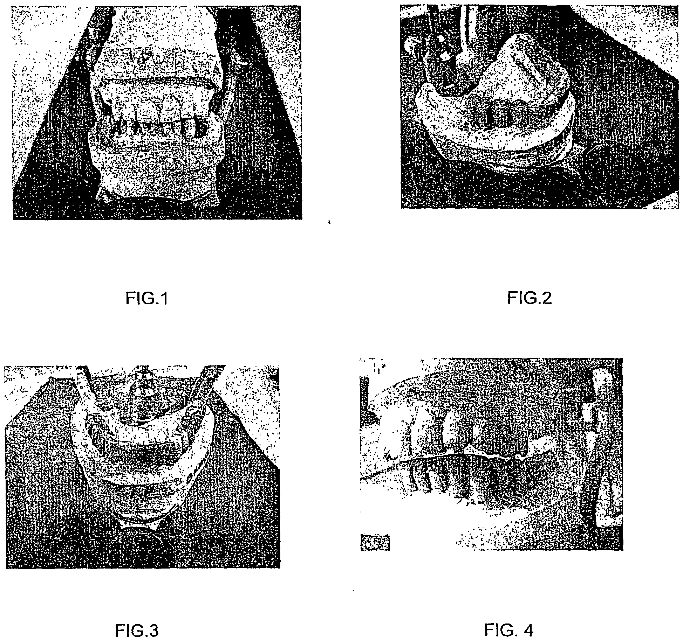

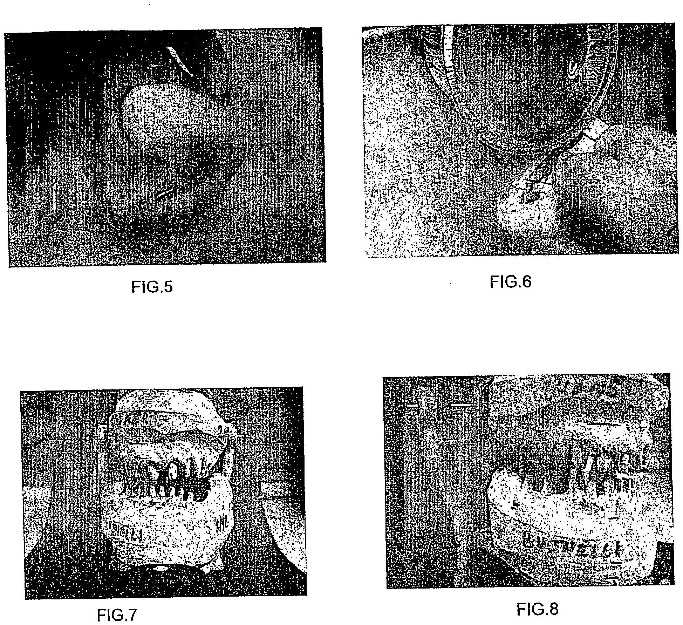

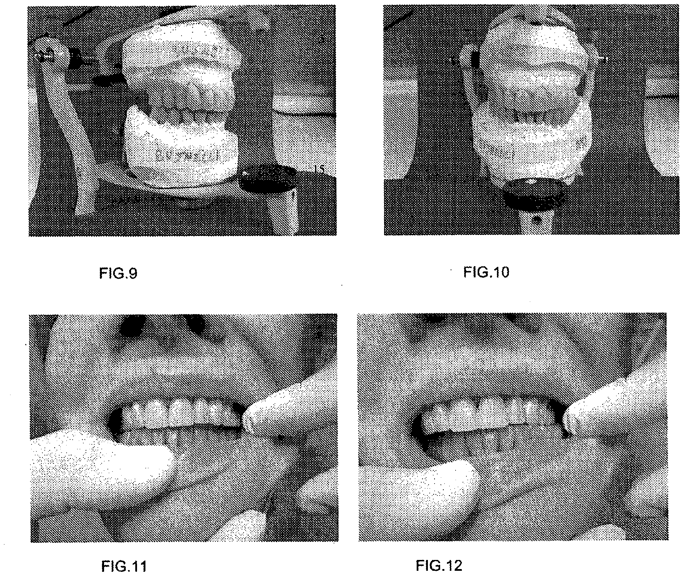

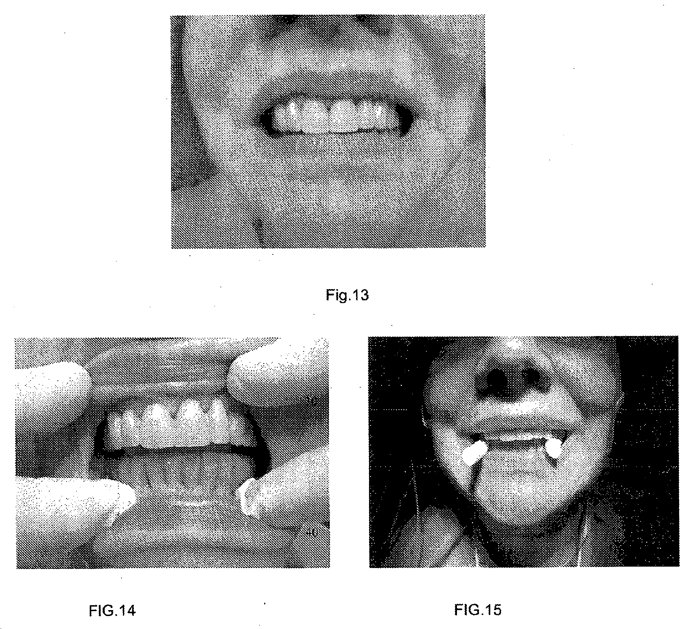

According to this invention the waxes are preferably used in the following operative phases in the preparation of crowns or dental bridges,
a) Before beginning rehabilitative work in the mouth of a patient, the dentist takes two impressions in alginate of the antagonist arches; these impressions will be filled with plaster in the dental technician's laboratory, to recreate two models for the upper arch and two models for the lower arch, called "study models" or "preliminary treatment models". These two models will be used for a diagnostic modelling, in other words an initial rehabilitation study to present to the patient (see Figures 1-4).
b) after having decided which treatment to carry out, the dentist asks the dental technician's laboratory to prepare the provisional shelled teeth which are polymerized on the models obtained previously during stage (a),
c) the provisional shells are then made in the following way:
   (c1) a silicone template is applied to the teeth that are to undergo prosthesis,
   (c2) the next stage is to shave down into stumps the plaster reproduction of the teeth that are to undergo prosthesis and
   (c3) with the help of the silicone template prepared in (c1), the teeth are polymerized with resin on the aforementioned stumps prepared in (c2), and are then perfected and polished.
d) once the provisional shelled teeth have been removed from the model, the silicone template is placed into position, into which the wax, the subject of this invention, is poured in a liquid state; once the wax has solidified and cooled, it is extracted from the mask and archived with the aforementioned preliminary treatment models obtained in (a), and the provisional shelled teeth obtained in (b) are repositioned on the plaster mould; said shelled teeth must be assessed by the dentist for the cementing which follows (see figures 5 and 6)
e) next, further provisional teeth are prepared, called "second provisionals" or "last provisionals"; these are made based on the stumps previously prepared by the dentist in the patient's mouth, according to the following procedures
   (e1) two alginate impressions are taken of the teeth that have been shaved down into stumps; the impressions are then developed in plaster at the laboratory, and the plaster casts obtained in this way are mounted on an articulator with the help of a centric wax and a facial arch (see figures 7 and 8).
   (e2) the blue wax that we had previously archived is placed on the plaster stumps, and we perfect the modelling and send it to the dental clinic for testing, (see figures 9 and 10),
   (e3) the dentist will position the wax on the stumps in the patient's mouth and will carry out all checks, including aesthetics, shape, volume, inclinations, phonetics, mastication, height of the teeth that we have modelled; any necessary modifications are made directly at the dental clinic, since wax is a material that can be easily manipulated; another check is made in the mouth to verify whether the results are satisfactory for the dentist, if so the wax model returns to the dental technician's laboratory and is used to carry out the polymerization process of the second provisional teeth (see figures 11-16).
   (e4) the next step is to construct the silicone template on the wax, according to the present invention the wax is removed from the stumps and is once again archived with the preliminary treatment models;
   (e5) the provisional teeth are then polymerized on the silicone template, and lastly they are perfected and polished, and are sent to the clinic to be cemented in the mouth. Wax modified in this way has all the characteristics that are required in the definitive article and also has a further advantage in that, during the test in the mouth, it allows the dentist to correct any errors in the preparation of the stumps (crookedness, inclination, length).

Having already decided what the final height of the prosthesis will be, allows a resin rest or gig (mastication guide) to be prepared in the laboratory; this will be used for further mounting of casts on the articulators. This will allow us to proceed used for further mounting of casts on the articulators. This will allow us to proceed safely with the construction of the prosthesis.

The use of the wax according to this invention is also of interest with the PRESS OVER and PRESSABLE CERAMIC SYSTEM technique, in which the aesthetic material, in other words the ceramic, is not modelled by hand and fired on the metal framework but instead is die-cast in a special oven with the lost wax technique.

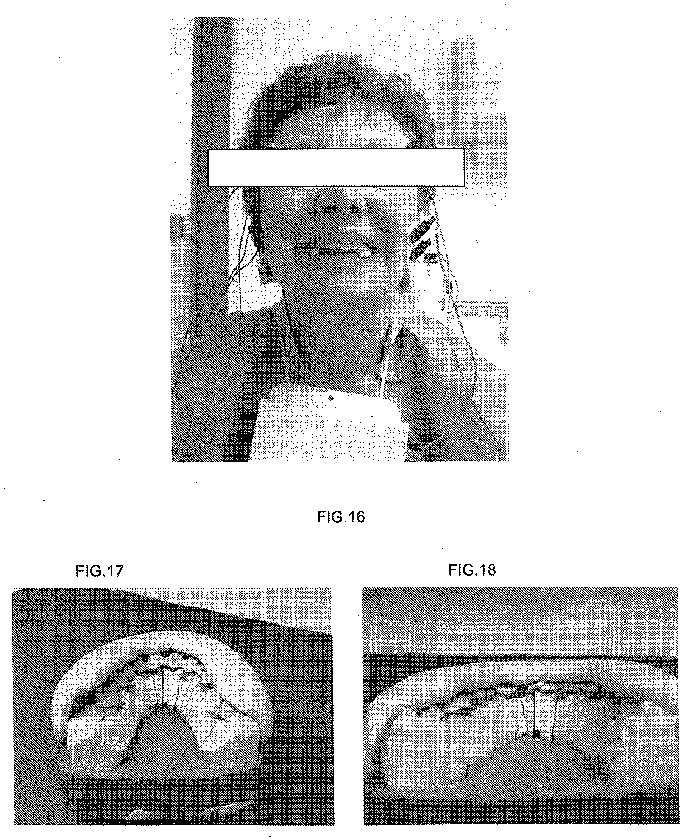

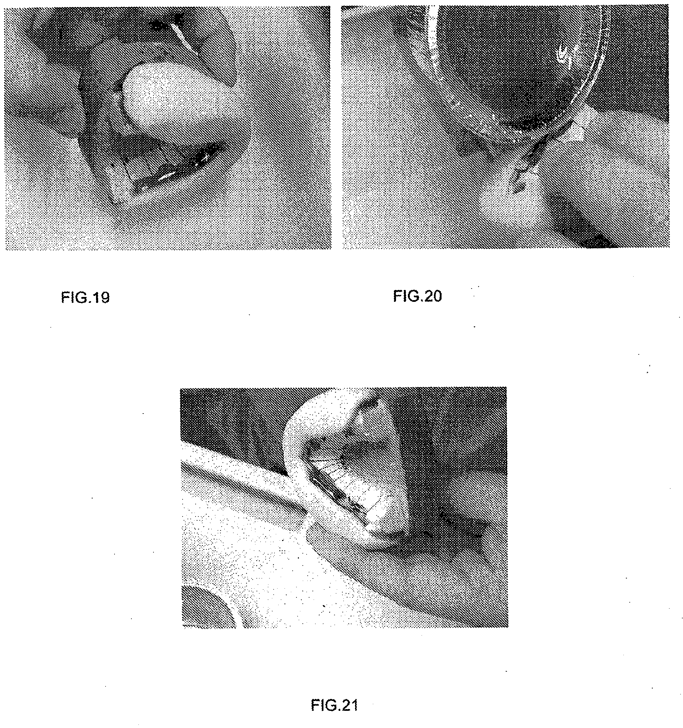

With this technique, the wax according to this invention can be used in the following way:
(a') after having tested the wax in the mouth as described in (e), the "master" models are made, on which the prosthesis work will be carried out. After having prepared the stumps, the functionalized diagnostic wax casts according to this invention are positioned and the silicone templates are arranged, to be used to prepare the metal framework. The masks will then be used for the shaping, over the bridge of the teeth, which will simply be performed by pouring liquid wax into the mask. Once the waxes according to this invention have cooled, they allow a modelling over the metal to be achieved which has an identical shape and size to that tested in the mouth and judged to be optimal by the dentist (see figures 17-21).
(b') the wax according to this invention is poured over the metal into the silicone template, and when the wax has solidified the silicone template is removed, thus obtaining teeth with an identical shape to those tried in the mouth and judged to be optimal by the dentist.
(c') then, using the lost wax fusion technique, the wax is replaced with pressure-cast ceramic in a special oven.

The waxes to be used according to the present invention are those that are habitually used in dental mechanics, such as those of a natural type for example, including mineral waxes and in particular paraffin waxes, vegetable waxes and waxes of animal-origin, or synthetic waxes. In any case, the critical element that is necessary for these waxes to be directly used in the patient's mouth is that they have a melting temperature, penetration and kinematic viscosity within the aforementioned recommended intervals. Preferably, the waxes that are the object of this invention should have needle penetration measured according to standard.

ASTM D1321 included between 9 and 14 dmm (tenths of mm), kinematic viscosity measured at 99°C according to standard ASTM D445 between 7 and 15 mm²/s. Even more preferable is that the waxes that are the object of this invention should present almost no shrinkage or contraction following solidification.

Almost no shrinkage following solidification means a contraction in volume following solidification that is lower than 11%, preferably lower than 5%, and even more preferably lower than 2%.

Preferably, the waxes that fit the abovementioned requirements are waxes that contain paraffinic hydrocarbons. Even more preferably, these waxes should contain at least two of the following hydrocarbons C₂₆H₅₄, C₂₇H₅₆, C₂₈H₅₈, C₂₉H₆₀, C₃₀H₆₂, C₃₁H₆₄ each at concentrations higher than 10% by weight of the total weight of the hydrocarbons, and the total concentration of these hydrocarbons compared to the total weight of said hydrocarbons should be between 45 and 65% by weight out of the total weight of hydrocarbons contained in the wax, and moreover, the principal hydrocarbon should be C₂₉H₆₀.

A particularly favourable embodiment of wax to use according to this invention is wax (2) containing C₂₆H₅₄, C₂₇H₅₆, C₂₈H₅₈, C₂₉H₆₀, each in concentrations of over 10% and the total concentration of these hydrocarbons is around 47% of the total weight of hydrocarbons contained in the wax.

Another particularly favourable embodiment to use according to this invention is wax (2A) containing C₂₇H₅₆, C₂₈H₅₈, C₂₉H₆₀, C₃₀H₆₂ in concentrations of over 10%, where the total concentration of said hydrocarbons is 46% of the total hydrocarbons contained in the wax.

Another preferred embodiment is wax (3) which contains C₂₇H₅₆, C₂₈H₅₈, C₂₉H₆₀, C₃₀H₆₂ , C₃₁H₆₄ each in a concentration of over 10% by weight, and concentrated so that the total concentration of these hydrocarbons is around 62% of the total weight of hydrocarbons in the wax.

The preferred waxes to be used according to this invention are in turn preferably obtained by blending at least two of the aforementioned waxes:
Wax (A) containing hydrocarbons C₂₆H₅₄, C₂₇H₅₆, C₂₈H₅₈, C₂₉H₆₀, C₃₀H₆₂ C₃₁H₆₄ each at a concentration higher than 10% by weight and so that the total concentration is around 71% of the total weight of hydrocarbons in the wax, this wax is available on the market under the trade name ELASTODIP.
Wax (B), in which the hydrocarbons, each of which are present in amounts higher than 10% by weight out of the total weight of hydrocarbons, are C₂₈H₅₈, C₂₉H₆₀ , C₃₀H₆₂ , C₃₁H₆₄,C₃₂H₆₆, and are present in amounts totalling around 70% of the total weight of the hydrocarbons; this wax is available on the market under the trade name Solidus 84.
Wax (C) in which the hydrocarbons, each present in quantities higher than 10%, are C₂₇H₅₆, C₂₈H₅₈, C₂₉H₆₀, and the total concentration of said hydrocarbons compared to the total amount of hydrocarbons in the wax is around 35%. This wax is available on the market under the name Corning's.

However, the Applicant has found that none of these commercial waxes can be used alone for the purposes of this invention, as it is not possible to obtain with them wax models of teeth that can be directly applied to the mouth.

Even more favourably, the waxes to use according to this invention should be prepared by blending wax (A) with at least one of the abovementioned waxes (B) and (C).

In a particularly favourable embodiment, the waxes to use according to this invention are prepared by blending wax (A) with wax (C) in the following ponderal ratios comprised between 1:6 and 1:2. This class includes, for example, wax (2) obtained by blending wax (A) and wax (C) in ponderal ratios of 1:6 and wax (2A) obtained by blending wax (A) and wax (C) in ponderal ratios of 1:2.

In another embodiment that is particularly favourable, the waxes to use according to this invention are obtained by blending wax (A) in quantities between 55 and 20% of the total weight of the blend, wax (B) in quantities between 20 and 45% of the total weight of the wax, and finally wax (C) in ratios between 40 and 60% by weight of the total weight of the blend. This class includes, for example, wax (3), which is obtained by mixing wax (A) in a quantity of around 15%, with wax (C) in a quantity of around 42% and wax (B) in the same percentage amount as (C).

The Applicant has also found that other waxes also meet the aforementioned requirements.

For example, this category includes waxes obtaining by blending at least 4 of the following waxes in the right way:
D) refined paraffin 52/54 with a melting point measuring according to standard ASTM D87: 51.5-54.0°C, kinematic viscosity at 100°C according to standard D445 3- 3,5 (mm²/s), needle penetration measured at 25°C (1/10mm) according to standard ASTM D1321: 14-20 dmm (1/10mm),
E) CW 82 is a mix of Vybar 103 paraffin wax , microcrystalline wax and a hydrocarbon resin, and has the following characteristics: melting point measured according to standard ASTM D566: 62-64°C, needle penetration measured at 25°C (1/10mm) according to standard ASTM D1321: 6-8 dmm (1/10mm),
F) Eltene LCS is a blend that includes paraffin and ethylene-vinylacetate copolymers, and has the following characteristics: solidification point measured according to standard ASTMD938: 78-80°C, penetration measured according to standard ASTM D1321 6-8 dmm (1/10mm), Brookfield viscosity measured at 150°C 800-900 cps, at 130°C 1400-1450cps, at 120°C 1900-1950 cps.
G) Licowax PE520 non-oxidized polyethylene wax with the following characteristics: drop melting point calculated with standard ASTM D127: 117-122°C, acid number calculated with standard ASTM D1386: 0 mg/KOH/g, saponification index calculated with standard ASTM D1387 0 mg/KOH/g, density at 20°C calculated with standard ASTM D1505 0,92-0,94 g/cm³
H) Vybar 103 wax with the following characteristics: softening point calculated according to modified standard ASTM D-36: 68,30-79,40°C, Brookfield viscosity at 210°C calculated according to modified standard ASTM D-3236: 250-440cps, needle penetration measured at 77°C with standard ASTM D-1321: 3-7 dmm, Even more preferably, the waxes according to this invention contain a blend of 4 of the abovementioned waxes. A particularly preferred embodiment of this type is that in which the blend is formed of the aforementioned waxes (D), (E), (F) and (H).

Other preferred embodiments are those that involve the use of blends containing all 5 of the aforementioned waxes. Even more preferably, these blends contain: wax (D) in quantities between 40 and 60% by weight of the total weight of the blend,
wax (E) in quantities between 30 and 40% by weight of the total weight of the blend,
wax (F) in quantities between 5 and 20% by weight of the total weight of the blend, wax (G) in quantities between 0 and 8% by weight of the total weight of the blend, wax (H) in quantities between 0.1 and 5% by weight of the total weight of the blend.

Below, for illustrative and non-limitative purposes, some examples are given of waxes obtained through blending wax (A) (Elastodip) with at least one of the waxes (B) (Solidus 84) and (C) (Corning's), as well as some examples of waxes obtained by blending waxes (D) (refined paraffin 52/54), E (CW 82), (F) (Eltene LCS), (G) Licowax PE520, (H) Vybar 103.

**TABLE 1 - COMPOSITION OF THE WAXES**

| | Wax (C) | Wax (A) | Wax (B) |
|---|---|---|---|
| | Corning's wax | Elastodip | Solidus 84 |
| Wax 1 | 30 g | 5 g | |
| Wax 2 | 30 g | 10 g | |
| Wax 2A | 30 g | 15 g | |
| Wax 3 | 25 g | 9 g | 25 g |
| Wax 4 | 35 g | 9 g | 15 g |
| Wax 5 | 30 g | 9 g | 20 g |

Waxes 2, 2A, 3 as well as commercial waxes (A), (B) and (C) were analysed with gas chromatography, using elicosane as a solvent, and identified through mass spectrometry.

For each of the abovementioned waxes, the % of the hydrocarburic components within them is given in table form.

**TABLE 2 - Hydrocarbon compositions of the waxes to be used according to the present invention and of commercial waxes**

| Wax 2 (%) hydr. | Wax 2A | Wax 3 | (A) Elastodip | (B) Solidus 84 | (C) Corning's wax | Number of carbons in hydr. |
|---|---|---|---|---|---|---|
| 2.8 | 0.2 | 3.8 | 0.1 | 0.0 | 0.31 | 21 |
| 1 | 0.8 | 0.6 | 0.4 | 0.2 | 1.0 | 22 |
| 3 | 3.1 | 1.8 | 1.1 | 0.7 | 3.5 | 23 |
| 6.4 | 5.6 | 4.7 | 3.5 | 2.1 | 6.1 | 24 |
| 8.8 | 8.1 | 6.6 | 6.3 | 3.9 | 8.3 | 25 |
| 10.2 | 9.2 | 8.6 | 10.1 | 6.2 | 9.0 | 26 |
| 12.5 | 11.9 | 11.2 | 12.6 | 8.3 | 11.3 | 27 |
| 11.6 | 11.7 | 11.6 | 12.6 | 10.9 | 11.2 | 28 |
| 13 | 12.6 | 14.4 | 13.1 | 14.3 | 12.02 | 29 |
| 9.8 | 10.1 | 13.4 | 11.7 | 17.1 | 9.8 | 30 |
| 8.9 | 9.3 | 11.0 | 10.6 | 15.6 | 9.1 | 31 |
| 6.2 | 6.9 | 7.0 | 7.5 | 11.3 | 7.1 | 32 |
| 4.9 | 5.2 | 4.2 | 5.8 | 6.0 | 5.4 | 33 |
| 2 | 3.2 | 1.3 | 3.5 | 2.6 | 3.5 | 34 |
| N.S. | 3.2 | N.S. | 0.7 | 0.7 | 2.2 | 35 |
| N.S. | 0.0 | N.S. | 0.5 | 0.0 | 0.1 | 36 |

**TABLE 3 - COMPOSITION OF WAXES**

| BASE WAXES USED | Wax 6 | Wax 7 | Wax 8 | Wax 9 |
|---|---|---|---|---|
| (D) Refined paraffin 52/54 | 200 g | 200g | 210g | 210g |
| (E) CW 82 | 140g | 140 g | 160g | 160g |
| (F) Eltene LCS | 27g | 30g | 80g | 80g |
| (G) Licowax PE 520 | 5g | 10g | 10g | |
| (H) VYBAR | 2g | 2g | 30g | 20g |

### Chemical physical properties of wax 8:

solidification point (ASTM D 938): 85-87°C
needle penetration (ASTM D1321) 9-10 dmm (1/10mm)

### Chemical physical properties of wax (9)

Solidification point (ASTM D 938): 60-62°C
Needle penetration (ASTM D1321): 10-12 dmm (1/10mm)
Kinematic viscosity (ASTM D445), 7.2-8.5 mm²/s

## Claims

1. Dental wax compositions containing at least two of the following paraffinic hydrocarbons C₂₆H₅₄, C₂₇H₅₆, C₂₈H₅₈, C₂₉H₆₀, C₃₀H₆₂, C₃₁H₆₄ each at concentrations higher than 10% by weight of the total weight of said hydrocarbons, and the total concentration of said hydrocarbons is between 45 and 65% by weight of the total weight of hydrocarbons contained in the wax, and furthermore the principal hydrocarbon is C₂₉H₆₀ and said composition showing:
□ solidification point between 60 and 90°C measured according to standard ASTM D938,
□ penetration measured according to standard ASTM D1321 between 8 and 20 dmm (tenths of mm),
□ a kinematic viscosity measured at 99°C according to standard ASTM D445 between 6 and 20 mm²/s.

2. Compositions according to claim 1 showing:
□ needle penetration measured according to standard ASTM D1321 between 9 and 14 dmm (tenths of mm),
□ kinematic viscosity measured at 99°C according to standard ASTM D445 between 7 and 15 mm²/s.

3. Compositions according to any of claims 1-2 showing almost no shrinkage or contraction following solidification.

4. Compositions according to claim 3 showing a contraction following solidification of less than 5%.

5. Compositions according to claim 4 showing a contraction following solidification of less than 2%.

6. Compositions according to claim 1 containing C₂₆H₅₄, C₂₇H₅₆, C₂₈H₅₈, C₂₉H₆₀, each at concentrations higher than 10%, and the total concentration of said hydrocarbons is 47% of the weight of hydrocarbons present in the wax.

7. Compositions according to claim 1 containing C₂₇H₅₆, C₂₈H₅₈. C₂₉H₆₀, C₃₀H₆₂ at concentrations higher than 10%, and the concentration of said hydrocarbons is around 46% of the total hydrocarbons present in the wax.

8. Compositions according to claim 1 containing C₂₇H₅₆, C₂₈H₅₈, C₂₉H₆₀, C₃₀H₆₂, C₃₁H₆₄, each at concentrations higher than 10% by weight and in such concentrations that the total concentration of said hydrocarbons is around 62% of the total weight of hydrocarbons present in the wax.

9. Model of teeth directly testable in a patient's oral cavity and directly functionalizable, said model consisting of a dental wax composition as defined in any of claims 1-8.

10. A process for preparing dental prosthesis said process **characterised in that**, during the preparation phase, a model as defined in claim 9 is directly tested in the patient's oral cavity and functionalized by the dentist thus obtaining an optimised model that will allow to produce an already optimised dental prosthesis; wherein said dental prosthesis is fixed and/or combined crowns or dental bridges on both natural or implanted pillars.

11. Process according to claim 10 wherein the dental prosthesis is prepared with in the PRESS OVER technique.

12. A process for preparing compositions of claims 1-8 by blending at least two of the following waxes:
| | |
|---|---|
| Wax (A) | containing hydrocarbons C₂₆H₅₄, C₂₇H₅₆, C₂₈H₅₈, C₂₉H₆₀, C₃₀H₆₂ C₃₁H₆₄ C₃₁H₆₄ each at a concentration higher than 10% by weight and in such concentrations that the total concentration of said hydrocarbons is around 71 %; |
| Wax (B), | in which the hydrocarbons present, each in quantities higher than 10% by weight of the total weight of the hydrocarbons, are C₂₈H₅₈, C₂₉H₆₀, C₃₀H₆₂, C₃₁H₆₄,C₃₂H₆₆ and are present in total quantities of around 70% of the total weight of hydrocarbons; |
| Wax (C) | in which the hydrocarbons present, each in quantities higher than 10%, are C₂₇H₅₆, C₂₈H₅₈, C₂₉H₆₀ and the total concentration of said hydrocarbons compared to the total amount of hydrocarbons present in the wax is around 35%. |

13. Process according to claim 12 wherein wax (A) is blended with at least one of the waxes (B) and (C).

14. Process according to claim 12 wherein wax (A) is blended with wax (C) in the following ponderal ratios between 1:6 and 1:2.

15. Process according to claim 12 wherein waxes are blended in the following quantities on the total weight of the blend:
wax (A) 15-20 wt%,
wax (B) ) 20-45 wt%, and
wax (C) 40-60 wt%.

16. A process for preparing compositions of claims 1-8 by blending at least 4 of the following waxes:
| | |
|---|---|
| wax (D) | refined paraffin 52/54 with melting point measured according to standard ASTM D87: 51.5-54.0°C, kinematic viscosity at 100°C according to standard ASTM D445 3- 3.5 (mm²/s), needle penetration measured at 25°C (1/10mm) according to standard ASTM D1321: 14-20 dmm (1/10mm); |
| wax (E) | blend of: |
| - | wax (H), |
| - | microcrystalline wax, |
| - | hydrocarbon resin, |
| | which shows the following properties: melting point measured according to standard ASTM D566: 62-64°C, needle penetration measured at 25°C (1/10mm) according to standard ASTM D1321: 6-8 dmm (1/10mm), |
| wax (F) | mix including paraffin and ethylene-vinylacetate copolymers which shows the following properties: solidification point measured according to standard ASTM D 938: 78-80°C, needle penetration measured according to standard ASTM D1321 6-8 dmm (1/10mm), Brookfield viscosity measured at 150°C 800-900 cps, at 130°C 1400-1450 cps, at 120°C 1900-1950 cps, |
| wax (G) | non-oxidized polyethylene wax with the following properties: drop melting point calculated with standard ASTM D127: 117-122°C, acidity number calculated with standard ASTM D1386: 0 mg/KOH/g, saponification index calculated with standard ASTM D1387 0 mg/KOH/g, density at 20°C calculated with standard ASTM D1505 0.92-0.94 g/cm³, |
| wax (H) | paraffin wax with the following properties: softening point measured according to modified standard ASTM D-36: 68.30-79.40°C, Brookfield viscosity at 210°C calculated according to modified standard ASTM D-3236: 250-440cps, needle penetration measured at 77°C with standard ASTM D-1321: 3-7 dmm. |

17. Process according to claim 16 wherein are blended 4 of waxes (D)-(H).

18. Process according to claim 17 wherein the 4 blended waxes are waxes (D), (E), (F) and (H).

19. Process according to claim 16 wherein are blended all 5 waxes (D)-(H).

20. Process according to any of claims 16-19 wherein waxes are blended in the following quantities on the total weight of the blend:
wax (D) 40-60 wt%,
wax (E) 30-40 wt%,
wax (F) 5-20 wt%,
wax (G) 0-8 wt%,
wax (H) 0.1-5 wt%.

## Patentansprüche

1. Zusammensetzungen für Zahnwachs, welche mindestens zwei der folgenden ParaffinKohlenwasserstoffe C₂₆H₅₄, C₂₇H₅₆, C₂₈H₅₈, C₂₉H₆₀, C₃₀H₆₂ und C₃₁H₆₄ enthalten, davon jeder mit einer Konzentration über 10 Gew.-%, bezogen auf das Gesamtgewicht der genannten Kohlenwasserstoffe, und bei welchen die Gesamtkonzentration der genannten Kohlenwasserstoffe zwischen 45 und 65 Gew.-%, bezogen auf das Gesamtgewicht der in dem Wachs enthaltenen Kohlenwasserstoffe, beträgt und außerdem der den Hauptbestandteil bildende Kohlenwasserstoffe C₂₉H₆₀ ist und die genannten Zusammensetzungen die folgenden Merkmale zeigen:
• Erstarrungspunkt zwischen 60 und 90 °C, gemessen nach Standard ASTM D-938;
• Eindringtiefe zwischen 8 und 20 dmm (Zehntel mm), gemessen nach Standard ASTM D-1321;
• kinematische Viskosität zwischen 6 und 20 mm²/s, gemessen bei 99 °C nach Standard ASTM D-445.

2. Zusammensetzungen nach Anspruch 1, welche die folgenden Merkmale zeigen:
• Nadeleindringtiefe zwischen 9 und 14 dmm (Zehntel mm) gemessen nach Standard ASTM D-1321
• kinematische Viskosität zwischen 7 und 15 mm²/s, gemessen bei 99 °C nach Standard ASTM D-445.

3. Zusammensetzungen nach einem der Ansprüche 1 und 2, welche nach dem Erstarren fast keine Schrumpfung oder Kontraktion zeigen.

4. Zusammensetzungen nach Anspruch 3, welche nach dem Erstarren eine Kontraktion von weniger als 5 % aufweisen.

5. Zusammensetzungen nach Anspruch 4, welche nach der Erstarren eine Kontraktion von weniger als 2 % aufweisen.

6. Zusammensetzungen nach Anspruch 1, welche C₂₆H₅₄, C₂₇H₅₆, C₂₈H₅₈ und C₂₉H₆₀ enthalten, davon jeder mit einer Konzentration über 10 %, und bei welchen die Gesamtkonzentration der genannten Kohlenwasserstoffe 47 % des Gewichts der im Wachs vorhandenen Kohlenwasserstoffe beträgt.

7. Zusammensetzungen nach Anspruch 1, welche C₂₇H₅₆, C₂₈H₅₈, C₂₉H₆₀ und C₃₀H₆₂ enthalten, davon jeder mit einer Konzentration über 10 %, und bei welchen die Konzentration der genannten Kohlenwasserstoffe ungefähr 46 % des Gewichts der im Wachs vorhandenen Gesamtkohlenwasserstoffe beträgt.

8. Zusammensetzungen nach Anspruch 1, welche C₂₇H₅₆, C₂₈H₅₈, C₂₉H₆₀, C₃₀H₆₂ und C₃₁H₆₄ enthalten, davon jeder mit einer Konzentration über 10 Gew.-%, und in solchen Konzentrationen, dass die Gesamtkonzentration der genannten Kohlenwasserstoffe ungefähr 62 % des Gesamtgewichts der im Wachs vorhandenen Kohlenwasserstoffe beträgt.

9. Modell von Zähnen, welches in der Mundhöhle eines Patienten auf direkte Weise getestet werden kann und auf direkte Weise in Funktion gesetzt werden kann, wobei das genannte Modell aus einer Zahnwachszusammensetzung besteht, wie sie in irgend einem der Ansprüche 1 bis 8 festgelegt worden ist.

10. Verfahren zur Vorbereitung von Zahnprothesen, wobei das genannte Verfahren **dadurch gekennzeichnet ist, dass** während der Vorbereitungsphase ein Modell, wie dieses im Anspruch 9 festgelegt ist, auf direktem Wege in der Mundhöhle des Patienten getestet wird und vom Zahnarzt dergestalt in Funktion gesetzt wird, dass er ein optimiertes Modell erhält, welches erlaubt, eine bereits optimierte Zahnprothese zu fertigen, bei welcher die genannte Zahnprothese und/oder damit verbundene Kronen oder Zahnbrücken sowohl auf natürlichen oder implantierten Pfeilern befestigt werden.

11. Verfahren nach Anspruch 10, bei welchem die Zahnprothese mit Hilfe der PRESS-OVER-Technik vorbereitet wird.

12. Verfahren zur Zubereitung der Zusammensetzungen der Ansprüche 1 bis 8 durch Mischen von mindestens zwei der folgenden Wachse:
| | |
|---|---|
| Wachs (A), | welches die Kohlenwasserstoffe C₂₆H₅₄, C₂₇H₅₆, C₂₈H₅₈, C₂₉H₆₀, C₃₀H₆₂, C₃₁H₆₄, und C₃₁H₆₄ enthält, davon jeder mit einer Konzentration über 10 Gew.-%, und in solchen Konzentrationen, dass die Gesamtkonzentration der genannten Kohlenwasserstoffe ungefähr 71 % beträgt. |
| Wachs (B), | bei welchem die Kohlenwasserstoffe, die jeweils in Mengen über 10 Gew.-% in Bezug auf das Gesamtgewicht der Kohlenwasserstoffe vorhanden sind, die Substanzen C₂₈H₅₈, C₂₉H₆₀, C₃₀H₆₂, C₃₁H₆₄ und C₃₂H₆₆ sind und diese in Gesamtmengen von ungefähr 70 % des Gesamtgewichts der Kohlenwasserstoffe vorliegen. |
| Wachs (C), | bei welchem die Kohlenwasserstoffe, die jeweils in Mengen über 10 % vorhanden sind, die Substanzen C₂₇H₅₆, C₂₈H₅₈ und C₂₉H₆₀ sind und die Gesamtkonzentration der genannten Kohlenwasserstoffe im Vergleich mit der Gesamtmenge der im Wachs vorhandenen Kohlenwasserstoffe ungefähr 35 % beträgt. |

13. Verfahren nach Anspruch 12, bei welchem das Wachs (A) mit mindestens einem der Wachse (B) und (C) gemischt wird.

14. Verfahren nach Anspruch 12, bei welchem das Wachs (A) mit Wachs (C) in den folgenden Gewichtsverhältnissen zwischen 1:6 und 1:2 gemischt wird.

15. Verfahren nach Anspruch 12, bei welchem Wachse in den folgenden Mengen, bezogen auf das Gesamtgewicht der Mischung, gemischt werden:
Wachs (A) 15-20 Gew.-%
Wachs (B) 20-45 Gew.-%
Wachs (C) 40 - 60 Gew.-%.

16. Verfahren zur Zubereitung der Zusammensetzungen der Ansprüche 1 - 8 durch Mischen von mindestens vier der folgenden Wachse:
| | |
|---|---|
| Wachs (D) | raffiniertes Paraffin 52/54 mit einem Schmelzpunkt zwischen 51,5 und 54,0 °C, gemessen nach dem Standard ASTM D-87, einer kinematischen Viskosität von 3 -3,5 mm²/s, gemessen bei 100 °C nach ASTM D-445, einer Nadeleindringtiefe von 14 - 20 dmm (Zehntel mm), gemessen bei 25 °C nach dem Standard ASTM D-1321; |
| Wachs (E) | eine Mischung aus: |
| | - Wachs (H), |
| | - mikrokristallinem Wachs, |
| | - Kohlenwasserstoffwachs, |
| | welches die folgenden Merkmale zeigt: Schmelzpunkt 62 - 64 °C, gemessen nach dem Standard ASTM D-566; Nadeleindringtiefe von 6 - 8 dmm (Zehntel mm), gemessen bei 25 °C nach dem Standard ASTM D-1321; |
| Wachs (F) | eine Mischung, welche Paraffin und Ethylenvinylacatat-Kopolymere enthält, welche die folgenden Merkmale aufweist: Erstarrungspunkt 78 - 80 °C, gemessen nach Standard ASTM D-938; Nadeleindringtiefe von 6 - 8 dmm (Zehntel mm), gemessen bei 25 °C nach dem Standard ASTM D1321; Brookfield-Viskosität 800 - 900 cps bei 150 °C, 1400 - 1450 cps bei 130 °C und 1900 - 1950 cps bei 120 °C. |
| Wachs (G) | ein nicht oxidiertes Polyethylenwachs mit den folgenden Merkmalen: Tropfschmelzpunkt 117 - 122 °C berechnet nach Standard ASTM D-127; Säuregrad 0 mg KOH/g berechnet nach Standard ASTM 1386; Verseifungszahl 0 mg KOH/g, berechnet nach Standard ASTM D-1387; Dichte bei 20 °C 0,92 - 0,94 g/cm³, berechnet nach ASTM D-1505. |
| Wachs (H) | Paraffinwachs mit den folgenden Merkmalen: Erweichungspunkt 68,30 - 79,40 °C, gemessen nach dem abgeänderten Standard ASTM D-36;, Brookfield-Viskosität bei 210 °C 250 - 440 cps, berechnet nach dem abgeänderten Standard ASTM D-3236; Nadeleindringtiefe 3 - 7 dmm, gemessen bei 77 °C nach Standard ASTM D-1321. |

17. Verfahren nach Anspruch 16, bei welchem vier der Wachse (D) - (H) gemischt werden.

18. Verfahren nach Anspruch 17, bei welchem die vier gemischten Wachse die Wachse (D), (E), (F) und (H) sind.

19. Verfahren nach Anspruch 16, bei welchem alle 5 Wachse (D) - (H) gemischt werden.

20. Verfahren nach irgend einem der Ansprüche 16 - 19, bei welchem die Wachse in den folgenden Mengen, bezogen auf das Gesamtgewicht der Mischung, gemischt werden:
Wachs (D) 40 - 60 Gew.-%,
Wachs (E) 20 - 40 Gew.-%,
Wachs (F) 5 - 20 Gew.-%,
Wachs (G) 0 - 8 Gew.-%,
Wachs (H) 0,1 - 5 Gew.-%.

## Revendications

1. Compositions de cire dentaire contenant au moins deux des hydrocarbures paraffiniques suivants C₂₆H₅₄, C₂₇H₅₆, C₂₈H₅₈, C₂₉H₆₀, C₃₀H₆₂, C₃₁H₆₄ chacun à des concentrations plus élevées que 10 % en poids du poids total desdits hydrocarbures, et la concentration totale desdits hydrocarbures est comprise entre 45 et 65 % en poids du poids total des hydrocarbures contenus dans la cire, et de plus l'hydrocarbure principal est C₂₉H₆₀ et ladite composition présente :
- un point de solidification entre 60 et 90 °C mesuré selon la norme ASTM D938,
- une pénétration mesurée selon la norme ASTM D1321 entre 8 et 20 dmm (dixièmes de mm),
- une viscosité cinématique mesurée à 99 °C selon la norme ASTM D445 entre 6 et 20 mm²/s.

2. Compositions selon la revendication 1, présentant :
- une pénétration d'aiguille mesurée selon la norme ASTM D1321 entre 9 et 14 dmm (dixièmes de mm),
- une viscosité cinématique mesurée à 99 °C selon la norme ASTM D445 entre 7 et 15 mm²/s.

3. Compositions selon l'une quelconque des revendications 1 à 2, ne présentant presque pas de rétrécissement ou contraction après solidification.

4. Compositions selon la revendication 3, présentant une contraction après solidification de moins de 5 %.

5. Compositions selon la revendication 3, présentant une contraction après solidification de moins de 2 %.

6. Compositions selon la revendication 1, contenant C₂₆H₅₄, C₂₇H₅₆ C₂₈H₅₈, C₂₉H₆₀, chacun à des concentrations plus élevées que 10 %, et la concentration totale desdits hydrocarbures est de 47 % du poids des hydrocarbures présents dans la cire.

7. Compositions selon la revendication 1, contenant C₂₇H₅₆, C₂₈H₅₈, C₂₉H₆₀, C₃₀H₆₂ à des concentrations plus élevées que 10 %, et la concentration desdits hydrocarbures est d'environ 46 % des hydrocarbures totaux présents dans la cire.

8. Compositions selon la revendication 1, contenant C₂₇H₅₆, C₂₈H₅₈, C₂₉H₆₀, C₃₀H₆₂, C₃₁H₆₄, chacun à des concentrations plus élevées que 10 % en poids, et à des concentrations telles que la concentration totale desdits hydrocarbures est d'environ 62 % du poids total des hydrocarbures présents dans la cire.

9. Modèle de dents pouvant être directement mis à l'essai dans une cavité orale d'un patient et pouvant être directement fonctionnalisé, ledit modèle consistant en une composition de cire dentaire telle que définie dans l'une quelconque des revendications 1 à 8.

10. Procédé de préparation d'une prothèse dentaire, ledit procédé étant **caractérisé en ce que**, pendant la phase de préparation, un modèle tel que défini dans la revendication 9 est directement mis à l'essai dans la cavité orale d'un patient et fonctionnalisé par le dentiste obtenant ainsi un modèle optimisé qui permettra de produire une prothèse dentaire déjà optimisée ; dans lequel ladite prothèse dentaire est des couronnes ou bridges fixes et/ou combinés sur des piliers à la fois naturels ou implantés.

11. Procédé selon la revendication 10, dans lequel la prothèse dentaire est préparée avec la technique de surpressée (PRESS OVER).

12. Procédé de préparation de compositions des revendications 1 à 8, par mélange d'au moins deux des cires suivantes :
cire (A) contenant des hydrocarbures C₂₆H₅₄, C₂₇H₅₆, C₂₈H₅₈, C₂₉H₆₀, C₃₀H₆₂, C₃₁H₆₄ C₃₁H₆₄ chacun à une concentration plus élevée que 10 % en poids et à des concentrations telles que la concentration totale desdits hydrocarbures est d'environ 71 % ;
cire (B), dans laquelle les hydrocarbures présents, chacun dans des quantités plus élevées que 10 % en poids du poids total des hydrocarbures, sont C₂₈H₅₈, C₂₉H₆₀, C₃₀H₆₂, C₃₁H₆₄, C₃₂H₆₆ et sont présents dans des quantités totales d'environ 70 % du poids total des hydrocarbures ;
cire (C) dans laquelle les hydrocarbures présents, chacun à des quantités plus élevées que 10 %, sont C₂₇H₅₆, C₂₈H₅₈, C₂₉H₆₀ et la concentration totale desdits hydrocarbures par rapport à la quantité totale des hydrocarbures présents dans la cire est d'environ 35 %.

13. Procédé selon la revendication 12, dans lequel la cire (A) est mélangée avec au moins l'une des cires (B) et (C).

14. Procédé selon la revendication 12, dans lequel la cire (A) est mélangée avec la cire (C) dans les rapports pondéraux suivants compris entre 1:6 et 1:2.

15. Procédé selon la revendication 12, dans lequel les cires sont mélangées dans les quantités suivantes sur le poids total du mélange :
cire (A) 15 à 20 % en poids,
cire (B) 20 à 45 % en poids, et
cire (C) 40 à 60 % en poids.

16. Procédé de préparation des compositions des revendications 1 à 8 par mélange d'au moins 4 des cires suivantes :
cire (D) paraffine raffinée 52/54 avec point de fusion mesuré selon la norme ASTM D87 : 51,5 à 54,0 °C, viscosité cinématique à 100 °C selon la norme ASTM D445 3 à 3,5 (mm²/s), pénétration d'aiguille mesurée à 25 °C (1/10 mm) selon la norme ASTM D1321 : 14 à 20 dmm (1/10 mm) ;
cire (E) mélange de :
- cire (H),
- cire microcristalline,
- résine d'hydrocarbure,
qui présente les propriétés suivantes : point de fusion mesuré selon la norme ASTM D566 : 62 à 64 °C, pénétration d'aiguille mesurée à 25 °C (1/10 mm) selon la norme ASTM D1321 : 6 à 8 dmm (1/10 mm),
cire (F) mélange incluant de la paraffine et des copolymères d'éthylène-acétate de vinyle qui présente les propriétés suivantes : point de solidification mesuré selon la norme ASTM D938 : 78 à 80 °C, pénétration d'aiguille mesurée selon la norme ASTM D1321 6 à 8 dmm (1/10 mm), viscosité Brookfield mesurée à 150 °C de 800 à 900 cps, à 130 °C de 1 400 à 1 450 cps, à 120 °C de 1 900 à 1 950 cps,
cire (G) cire de poly(éthylène) non oxydé ayant les propriétés suivantes : point de fusion de goutte calculé avec la norme ASTM D127 : 117 à 122 °C, indice d'acidité calculé avec la norme ASTM D1386 : 0 mg/KOH/g, indice de saponification calculé avec la norme ASTM D1387 0 mg/KOH/g, masse volumique à 20 °C calculée avec la norme ASTM D1505 0,92 à 0,94 g/cm³,
cire (H) cire de paraffine ayant les propriétés suivantes : point de ramollissement mesuré selon la norme modifiée ASTM D-36 : 68,30 à 79,40 °C, viscosité Brookfield à 210 °C calculée selon la norme modifiée ASTM D-3236 : 250 à 440 cps, pénétration d'aiguille mesurée à 77 °C avec la norme ASTM D-1321 : 3 à 7 dmm.

17. Procédé selon la revendication 16, dans lequel 4 des cires (D) à (H) sont mélangées.

18. Procédé selon la revendication 17, dans lequel les 4 cires mélangées sont les cires (D), (E), (F) et (H).

19. Procédé selon la revendication 16, dans lequel les 5 cires (D) à (H) sont toutes mélangées.

20. Procédé selon l'une quelconque des revendications 16 à 19, dans lequel les cires sont mélangées dans les quantités suivantes sur le poids total du mélange :
cire (D) 40 à 60 % en poids,
cire (E) 30 à 40 % en poids,
cire (F) 5 à 20 % en poids,
cire (G) 0 à 8 % en poids,
cire (H) 0,1 à 5 % en poids.
